## Europäisches Patentamt
## European Patent Office
## Office européen des brevets

(11) Veröffentlichungsnummer: **0 207 402**
**A2**

(12) # EUROPÄISCHE PATENTANMELDUNG

(21) Anmeldenummer: **86108436.6**

(22) Anmeldetag: **20.06.86**

(51) Int. Cl.⁴: **C 12 N 15/00**
**C 12 P 21/00, C 12 N 9/76**

(30) Priorität: **03.07.85 DE 3523701**

(43) Veröffentlichungstag der Anmeldung:
**07.01.87 Patentblatt 87/2**

(84) Benannte Vertragsstaaten:
**AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Schmidt-Kastner, Günter, Prof.**
**Falkenberg 59**
**D-5600 Wuppertal 1(DE)**

(72) Erfinder: **Kutzbach, Carl, Dr.**
**3311 East Lake Drive North**
**Elkhart Indiana 46515(US)**

(72) Erfinder: **Wehlmann, Hermann, Dr.**
**Mastweg 3a**
**D-5600 Wuppertal 12(DE)**

(54) **Verfahren zur Herstellung von Proteinen und Polypeptiden.**

(57) Die Erfindung betrifft ein Verfahren zur Herstellung von Proteinen durch Coclonierung von DNA-Sequenzen, die ein biospezifisches Protein oder Polypeptid (I) und ein weiteres Protein (II), welches das herzustellende Protein oder ein Markerprotein codieren, wobei diese beiden DNA-Sequenzen über eine DNA-Sequenz verknüpft sind, die ein kurzkettiges Peptid (III) codiert, welches als spezifische Spaltungsstelle fungiert und dieses Peptid zusätzlich zur Spaltsequenz noch eine oder mehrere Aminosäuren enthält, die die Spaltungsspezifität und/oder Spaltungsgeschwindigkeit erhöhen. Dieses neue DNA-Stück wird mittels geeigneter gentechnologischer Methoden in einen Wirtsorganismus eingebracht, damit die Transkription, Translation und Expression erfolgen können. Das so erhaltene kombinierte Protein wird affinitätschromatographisch gereinigt und anschließend enzymatisch gespalten.

EP 0 207 402 A2

- 1 -

BAYER AKTIENGESELLSCHAFT          5090 Leverkusen, Bayerwerk
Konzernverwaltung RP
Patentabteilung                   Bu/ABc

## Verfahren zur Herstellung von Proteinen und Polypeptiden

Die Erfindung betrifft ein Verfahren zur Herstellung von
Proteinen durch Coclonierung von DNA-Sequenzen, die ein
biospezifisches Protein oder Polypeptid (I) und ein weiteres Protein (II), welches das herzustellende Protein
oder ein Markerprotein ist, codieren, wobei diese beiden DNA
Sequenzen über eine DNA-Sequenz verknüpft sind, die ein
kurzkettiges Peptid (III) codiert, welches als spezifische
Spaltungsstelle fungiert und dieses Peptid zusätzlich zur
Spaltsequenz noch eine oder mehrere Aminosäuren enthält,
die die Spaltungsspezifität und/oder Spaltungsgeschwindigkeit erhöhen. Dieses neue DNA-Stück wird mittels geeigneter gentechnologischer Methoden in einen Wirtsorganismus
eingebracht, damit die Transkription, Translation und
Expression erfolgen können. Das so erhaltene kombinierte
Protein wird affinitätschromatographisch gereinigt und
anschließend enzymatisch gespalten.

Bei der gentechnologischen Herstellung von Proteinen ist

Le A 23 922-Ausland

es stets ein Problem, das exprimierte Protein aus einem Vielstoff-Gemisch in reiner Form zu isolieren.

Zur Zeit bekannt ist die Coclonierung von Proteinen und Polypeptiden mit sogenannten Markerproteinen. Diese Markerproteine haben die Aufgabe, die Expression des gewünschten Proteins oder Polypeptids zu ermöglichen und den Nachweis des exprimierten Produktes zu erleichtern. Die Kopplung des Markerproteins mit dem gewünschten Protein erfolgt dabei über die Aminosäure Methionin. Durch den Einsatz von Bromcyan ist diese Bindung leicht spaltbar. Diese Methode hat aber den Nachteil, daß alle Bindungen, an denen Methionin beteiligt ist, gespalten werden, d.h. wenn das herzustellende Protein an einer oder mehreren Stellen Methionin enthält, wird es durch die Spaltung zerstört.

Mit dieser Erfindung wird ein Verfahren zur Verfügung gestellt, welches eine wesentliche Vereinfachung der gentechnischen Herstellung und Gewinnung von Proteinen und Polypeptiden darstellt.

Die Erfindung betrifft also ein Verfahren zur Herstellung eines Proteins, wobei man

a) die DNA-Sequenz für ein biospezifisches Protein oder Polypeptid (I) und die DNA-Sequenz für ein weiteres Protein (II), welches das herzustellende Protein oder ein Markerprotein ist, über eine DNA-Sequenz, die ein kurzkettiges Peptid (III) codiert, welches als spezifische Spaltungsstelle dient, verknüpft;

Le A 23 922

b) das so erhaltene neue DNA-Stück mittels geeigneter gentechnologischer Methoden so in die DNA eines Wirtsorganismus einbringt, daß die Expression eines kombinierten Proteins erfolgen kann, das aus mehreren Peptiden besteht, entsprechend den clonierten DNA-Sequenzen;

c) den Wirtsorganismus so kultiviert, daß er das kombinierte Protein erzeugt;

d) den das kombinierte Protein enthaltenden Teil des Kultivierungssystems, gegebenenfalls nach Abtrennen anderer Bestandteile, mit einem immobilisierten System in Kontakt bringt, das aus einem geeigneten Trägermaterial besteht, mit dem eine Substanz verbunden ist, die zu dem biospezifischen Protein oder Polypeptid komplementär ist und mit ihm unter den gegebenen Reaktionsbedingungen eine starke Bindung eingeht;

e) das immobilisierte System, an das das kombinierte Protein gebunden hat, in geeigneter Weise von übrigen Bestandteilen trennt und mit Hilfe von einem oder mehreren Enzymen spezifisch spaltet,

f) das biospezifische Protein oder Polypeptid (I) von dem Protein (II) abtrennt und das jeweils gewünschte gewinnt,

wobei das kurzkettige Peptid (III) zusätzlich zur Spaltsequenz noch eine oder mehrere Aminosäuren enthält, die die Spaltungsspezifität und/oder die Spaltungsgeschwindigkeit erhöhen.

Die Spaltsequenz ist eine von einem proteolytischen Enzym spezifisch erkennbare Aminosäuresequenz, die durch die Enzymwirkung hydrolytisch gespalten werden kann. Sie hat die Funktion einer "Sollbruchstelle". Erfindungsgemäß ist jede Protease einsetzbar, die in der Lage ist, eine spezifische Spaltsequenz zu erkennen und zu spalten. Bevorzugt verwendet wird Kallikrein als hydrolytisches Enzym. Die korrespondierende Spaltsequenz ist das Kallidin oder ein Derivat davon.

Der Einsatz von Enzymen bei der Spaltung des kombinierten Proteins stellt einen wesentlichen Fortschritt dar. Aufgrund ihrer Substratspezifität sind sie zu hochspezifischen Spaltungen besonders geeignet. Weiterhin ermöglicht die Coclonierung und Coexpression eines biospezifischen Proteins oder Polypeptids (I) die einfache Abtrennung des Expressionsproduktes von Fremdproteinen. Wesentlich für die Verwendung von Enzymen bei der Spaltung ist der Einbau einer von Enzymen erkennbaren Spaltsequenz zwischen dem

Le A 23 922

biospezifischen Protein oder Polypeptid (I) und dem weiteren Protein (II), welches das herzustellende Protein oder ein Markerprotein sein kann. Durch den Einbau von einer oder mehreren Aminosäuren zusätzlich zur Spaltsequenz läßt sich die Spezifität und/oder die Geschwindigkeit der Spaltung wesentlich erhöhen. Das erfindungsgemäße Verfahren ist zur Herstellung eines Proteins (II), das mit dem biospezifischen Protein oder Polypeptid cocloniert und exprimiert wird, ebenso geeignet, wie zur Herstellung des biospezifischen Proteins oder Polypeptids (I) oder auch von beiden gleichzeitig. Je nachdem, welches Produkt das gewünschte ist, ergeben sich Variationen in der Aminosäuresequenz des coclonierten und als Verknüpfung dienenden Peptids (III).

Im folgenden wird das erfindungsgemäße Prinzip mit Kallikrein als Spaltenzym und Kallidin als Spaltsequenz beispielhaft erläutert. Biospezifisches Polypeptid ist dabei das Aprotinin, das mit Trypsin als komplementärer Substanz eine starke Bindung eingehen kann.

Kallikrein spaltet sein natürliches Substrat - natives Kininogen - gleichzeitig an zwei Stellen mit hoher Umsatzrate unter Freisetzung des Dekapeptids Kallidin.

Le A 23 922

Kininogen-Sequenz

---Ser-Leu-Met-Lys-Arg-Pro-Pro-Gly-Phe-Ser-Pro-Phe-Arg-Ser-

        ↑                                         ↑

                                     Val-Gln----

                      ⬇ Kallikrein

----Ser-Leu-Met-OH                        H-Ser-Val-Gln----

        H-Lys-Arg-Pro-Pro-Gly-Phe-Ser-Pro-Phe-Arg-OH

        Kallidin (Dekapeptid, Intermediär-Peptid)

Für die Effizienz der Spaltung durch Kallikrein sind die Aminosäure-Sequenzen in unmittelbarer Nachbarschaft der Schnittstellen von Bedeutung.

Es wurde nun gefunden, daß die Effizienz der Spaltung, d.h. Spezifität und Geschwindigkeit durch das Kallikrein dadurch erhöht werden kann, daß zwischen dem biospezifischen Polypeptid Aprotinin und dem kurzkettigen Peptid Kallidin zusätzlich ein Tripeptid eingebaut wird, welches in seiner Sequenz der Sequenz des Kininogens an der Spaltstelle entspricht oder ihr ähnlich ist.

Zwei unterschiedliche Typen von kombinierten Proteinen sind möglich, denn das Aprotinin kann unter Zwischenschaltung eines entsprechenden Tripeptids entweder an den C-Terminus oder an den N-Terminus des Kallidins gebunden sein. Die entstehenden Fusionsproteine haben folgendes Aussehen:

Le A 23 922

**Co-Clonierungsmöglichkeit A:**

Kallidin

H$_2$N-Aprotinin-A$_1$-A$_2$-A$_3$-Lys-Arg-Pro-Pro-Gly-Phe-Ser-Pro-Phe-Arg- gewünschtes Protein-COOH

insbesondere

H$_2$N-Aprotinin-Ser-Leu-Met-Lys-Arg-Pro-Pro-Gly-Phe-Ser-Pro-Phe-Arg- gewünschtes Protein-COOH

— 7 —

**Co-Clonierungsmöglichkeit B:**

H$_2$N-gewünschtes Protein-Lys-Arg-Pro-Pro-Gly-Phe-Ser-Pro-Phe-Arg-A$_1$'-A$_2$'-A$_3$'-Aprotinin-COOH

insbesondere

H$_2$N-gewünschtes Protein-Lys-Arg-Pro-Pro-Gly-Phe-Ser-Pro-Phe-Arg-Ser-Val-Gln-Aprotinin-COOH

- 8 -

Das in diesem Beispiel erläuterte neue Verfahren zur Herstellung eines Proteins besteht nun daraus, daß man

entweder
a)  eine DNA-Sequenz, die für Aprotinin codiert,
eine DNA-Sequenz, die für ein Tripeptid $H-A_1-A_2-A_3-OH$ codiert,
vorzugsweise für H-Ser-Leu-Met-OH
eine DNA-Sequenz, die für das Kallidin codiert und
eine DNA-Sequenz, die für das gewünschte Protein
codiert in der Reihenfolge der Auflistung miteinander
verknüpft

oder

eine DNA-Sequenz, die für das gewünschte Protein
codiert,
eine DNA-Sequenz, die für Kallidin codiert,
eine DNA-Sequenz, die für ein Tripeptid $H-A_1'-A_2'-A_3'-OH$ codiert, vorzugsweise für H-Ser-Val-Gln-OH und
eine DNA-Sequenz, die für Aprotinin codiert
in der Reihenfolge der Auflistung miteinander verknüpft;

b) das so erhaltene neue DNA-Stück mittels geeigneter
gentechnologischer Methoden so in die DNA eines Wirtsorganismus einbringt, daß die Expression eines kombinierten Proteins erfolgen kann, das aus mehreren
Peptiden besteht, entsprechend den clonierten DNA-
Sequenzen;

Le A 23 922

c) den Wirtsorganismus so kultiviert, daß er das kombinierte Protein erzeugt;

d) den das kombinierte Protein enthaltenden Teil des Kultivierungssystems, gegebenenfalls nach Abtrennen anderer Bestandteile, mit einem immobilisierten System in Kontakt bringt, das aus einem geeigneten Trägermaterial besteht, mit dem eine Substanz verbunden ist, die zu dem biospezifischen Polypeptid Aprotinin komplementär ist und mit ihm unter den gegebenen Reaktionsbedingungen eine starke Bindung eingeht;

e) das immobilisierte System, an das das kombinierte Protein gebunden hat, in geeigneter Weise von den übrigen Bestandteilen trennt und mit Kallikrein behandelt, das spezifisch das Kallidin aus dem gebundenen kombinierten Protein abspaltet und

f) das auf diese Weise freigesetzte gewünschte Protein in geeigneter Weise von den übrigen Bestandteilen trennt und gewinnt.

Die an diesem Beispiel erläuterte Erfindung stellt eine Verbesserung der Spaltungsspezifität des Fusionsproteins durch Kallikrein dar. Die Schnittstelle zwischen Kallidin und Aprotinin ist durch das eingeschobene Tripeptid ideal für Kallikrein. Die Eignung der Schnittstelle zwischen Kallidin und dem gewünschten Protein kann durch die Wahl einer der beiden Coclonie-

Le A 23 922

rungsmöglichkeiten beeinflußt werden. Falls die N-terminale Aminosäure-Sequenz des gewünschten Proteins für die Spaltungseffizienz des kombinierten Proteins durch Kallikrein besser geeignet ist als die C-terminale Aminosäure-Sequenz des gewünschten Proteins, so empfiehlt sich die oben gezeigte Coclonierungsmöglichkeit A, im umgekehrten Falle die Coclonierungsmöglichkeit B. Die Auswahl zwischen der C- und der N-terminalen Coclonierungsstrategie richtet sich also nach der für die Spaltung der Sollbruchstelle durch Kallikrein günstigeren Aminosäure-Sequenz des gewünschten Proteins.

Das nach diesem Beispiel coclonierte Aprotinin hat die Aufgabe, an eine trägergebundene, biospezifisch-komplementäre Substanz zu binden, damit der Träger zusammen mit dem daran gebundenen kombinierten Protein von übrigen Bestandteilen des Mediums getrennt werden kann. Daraus folgt, daß an das System Aprotinin/komplementäre Substanz die Anforderung zu stellen ist, daß unter den gegebenen Reaktionsbedingungen eine starke Bindung zwischen ihnen erfolgt.

Biologisch aktive Polypeptide, z.B. Enzyme, können biospezifisch mit biologisch wirksamen komplementären Substanzen, z.B. mit Inhibitoren Komplexe, z.B. Enzym-Inhibitor-Komplexe bilden.

Solche biospezifisch gebildeten Enzym-Inhibitor-Komplexe besitzen eine hohe Bindungsaffinität. Ihre Dissoziationskonstante ist dementsprechend klein.

Le A 23 922

Ein Beispiel ist der erfindungsgemäß bevorzugte Trypsin-Aprotinin-Komplex, der aus dem Enzym Trypsin und aus dem Enzyminhibitor Aprotinin gebildet wird.

Die Dissoziationskonstante $K_i$ dieses Komplexes liegt bei nur $6 \times 10^{-15}$ mol/l (pH = 8,0, t = 25°C), die Komplexbildungsgeschwindigkeit bei $t_{1/2}$ = 6,3 Sekunden. Der Trypsin-Aprotinin-Komplex wird dementsprechend sehr schnell gebildet und besitzt eine sehr hohe Stabilität unter den gegebenen Bedingungen. Die Stabilität des Trypsin-Aprotinin-Komplexes ist jedoch abhängig vom pH-Wert der Lösung und nimmt mit sinkendem pH-Wert ab. Bei einem pH-Wert von ca. 2,0 ist der Trypsin-Aprotinin-Komplex fast vollständig dissoziiert. Ein solches Verhalten ist im Rahmen der Erfindung besonders wünschenswert, weil hierüber leicht eine Regenerierung der trägergebundenen Komplementärsubstanz möglich ist. Diese kann dann erneut im Verfahren eingesetzt werden. Es ist ferner wünschenswert, daß die Bildungsgeschwindigkeit der Bindung zwischen dem biospezifischen Polypeptid und der trägergebundenen Komplementärsubstanz hoch ist. Auf diese Weise lassen sich mögliche Konkurrenzreaktionen der trägergebundenen Substanz mit anderen Stellen des kombinierten Polypeptids reduzieren.

Die biospezifische Adsorption erfolgt bevorzugt unter Verwendung von in Wasser unlöslichen Trägern, an die die biospezifisch-komplementäre Substanz gebunden ist. Diese Bindung kann nach den bekannten Methoden der Immobilisierung mit oder ohne Spacer-Moleküle erfolgen.

Die DNA-Sequenz der genannten Komponenten wird nach Methoden der Gentechnologie cocloniert und die so erhaltene DNA nach Methoden der Gentechnologie in die DNA eines Wirtsorganismus, z.B. in ein Plasmid, übertragen. Als Wirtsorganismus können geeignete Mikroorganismen genutzt werden, z.B. Bakterien, insbesondere E. coli, aber auch Bac. subtilis oder Hefen. Die Expression der coclonierten DNA erfolgt während der Kultivierung des Mikroorganismus. Ist das gewünschte Protein mit den coclonierten Peptiden in der Fermentationslösung enthalten, so werden die Zellen, z.B. durch Zentrifugieren oder durch Cross-flow-Filtration abgetrennt und das Kulturfiltrat wird zur Isolierung verwendet Ist das gewünschte Protein mit den coclonierten Peptiden in der Zelle selbst enthalten, so werden die Zellen separiert und aufgeschlossen und die Zellbruchstücke abgetrennt. Das Kulturfiltrat oder der Zellextrakt enthalten nicht nur das gewünschte coclonierte kombinierte Protein, sondern darüber hinaus eine Vielzahl weiterer Proteine, eine Vielzahl anderer Produkte, aber auch anhaftende Nährmedienbestandteile. Aus dieser komplex zusammengesetzten Lösung wird nun das gewünschte kombinierte Protein isoliert und gereinigt. Dies erfolgt dadurch, daß man die trägergebundene biospezifisch-komplementäre Substanz, z.B. das immobilisierte Trypsin zu dem Kulturfiltrat oder Extrakt gibt. Es bildet sich augenblicklich mit hoher Komplexbildungsgeschwindigkeit der wasserunlösliche Komplex aus den Komponenten: Träger + biospezifisch-komplementäre Substanz + kombiniertes Protein.

Le A 23 922

Die Zugabe der carriergebundenen biospezifisch-komplementären Substanz kann bei Zimmertemperatur, aber auch, bei der hohen Komplexbildungsgeschwindigkeit, bei niedrigen Temparaturen, z.B. bei +2°C erfolgen. Der wasserunlösliche Komplex kann von anderen Proteinen der Fermentationslösung und auch von allen anderen Verunreinigungen durch einfache Filtration abgetrennt werden.

Wird als biospezifisch-komplementäre Substanz ein proteolytisches Enzym, z.B. Trypsin, verwendet, so kann dieses vor der Zugabe zur Fermentationslösung mit einem niedermolekularen Inhibitor abgesättigt werden. Die Dissoziationskonstante des niedermolekularen Inhibitors muß jedoch größer sein als die des Aprotinins. Der niedermolekulare Inhibitor wird aufgrund der unterschiedlichen Dissoziationskonstanten bei Zugabe der Fermentationslösung von dem biospezifischen Polypeptid verdrängt. Als niedermolekulare Inhibitoren für das trägergebundene Trypsin können z.B. Benzamidin mit einer Dissoziationskonstanten von $18 \times 10^{-6}$ mol/l oder Phenylguanidin mit einer Dissoziatonskonstanten von $72 \times 10^{-6}$ mol/l verwendet werden.

Alternativ kann die trägergebundene, biospezifisch-komplementäre Substanz in eine Säule gefüllt werden. Durch die Säule wird dann das Kulturfiltrat oder der Zellextrakt gegeben. Auch hier wird ausschließlich das gewünschte kombinierte Protein über das biospezifische Polypeptid an den festen Träger gebunden, während alle anderen Proteine und alle anderen Verunreinigungen durch die Säule laufen. Das an den Carrier gebundene biospezifisch-komplementäre Polypeptid sollte möglichst vollständig mit dem

Le A 23 922

- 14 -                                      U207402

zu bindenden Komplex abgesättigt sein. Die Säule kann
durch Waschen mit Pufferlösungen oder durch Waschen mit
anderen geeigneten Lösungen von restlichen, anhaftenden
Verunreinigungen befreit werden. Bei einer hohen Komplexbildungsgeschwindigkeit kann die Säule auch bei tiefen
Temperaturen beschickt werden.

Schließlich wird durch Einwirkung eines spezifischen
Enzyms das am Träger gebundene kombinierte Protein aufgespalten, wobei z.B. Kallikrein ausschließlich die Peptidbindungen am Kallidin spaltet.

Das gewünschte Protein wird zusammen mit dem Kallidin eluiert und kann von diesem nach bekannten Methoden, z.B.
durch Molekularsiebchromatographie, abgetrennt werden.
Das gewünschte Protein wird so in reiner Form erhalten.
Der Vorzug in der Wahl eines kurzkettigen Peptids als
Sollbruchstelle im kombinierten Protein ist darin zu
sehen, daß nach der Spaltung des gewünschte Protein als
in der Regel großes Molekül von dem kleinen kurzkettigen
Peptid leicht trennbar ist.

Aprotinin und anhängendes Tripeptid bleiben über die komplementäre Substanz unter den Bedingungen der Elution
am Träger gebunden. Erst durch Wechsel, z.B. des pH-Wertes
einer Pufferlösung, z.B. auf einen Wert von 2-3, werden
Aprotinin und anhängendes Tripeptid abgelöst und aus der
Säule ausgewaschen. Die Säule kann dann nach weiteren
Waschvorgängen wieder mit Pufferlösungen auf einen geeigneten pH-Wert, bei Verwendung von Trypsin/Aprotinin auf
pH 8,0 eingestellt und für einen weiteren Versuch wiederverwendet werden.

Le A 23 922

- 15 -

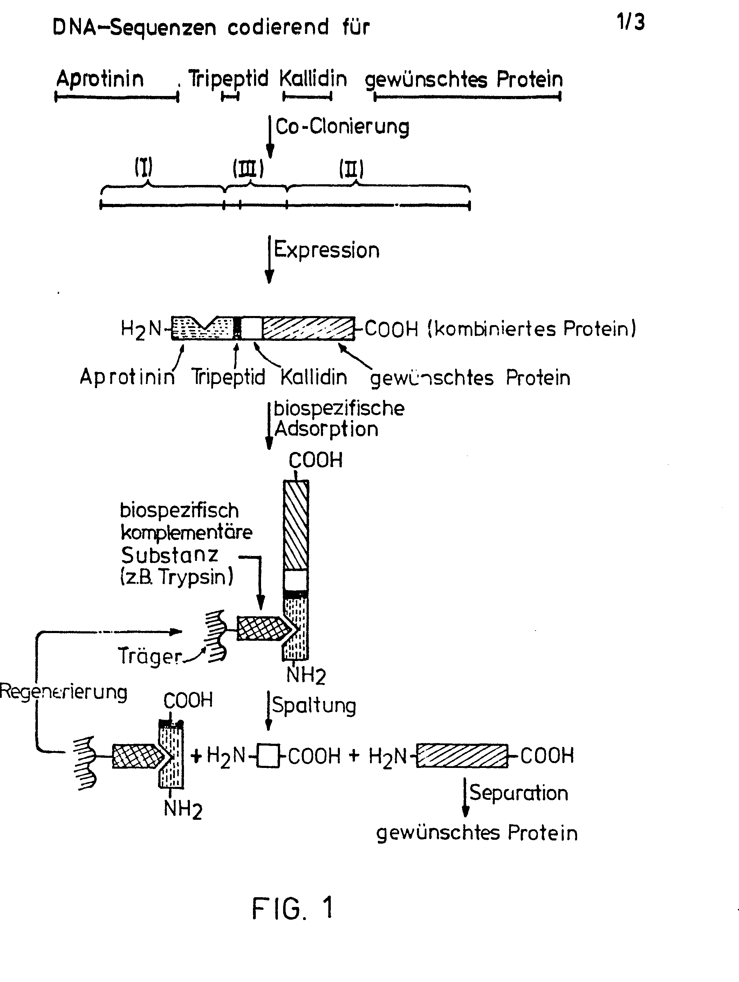

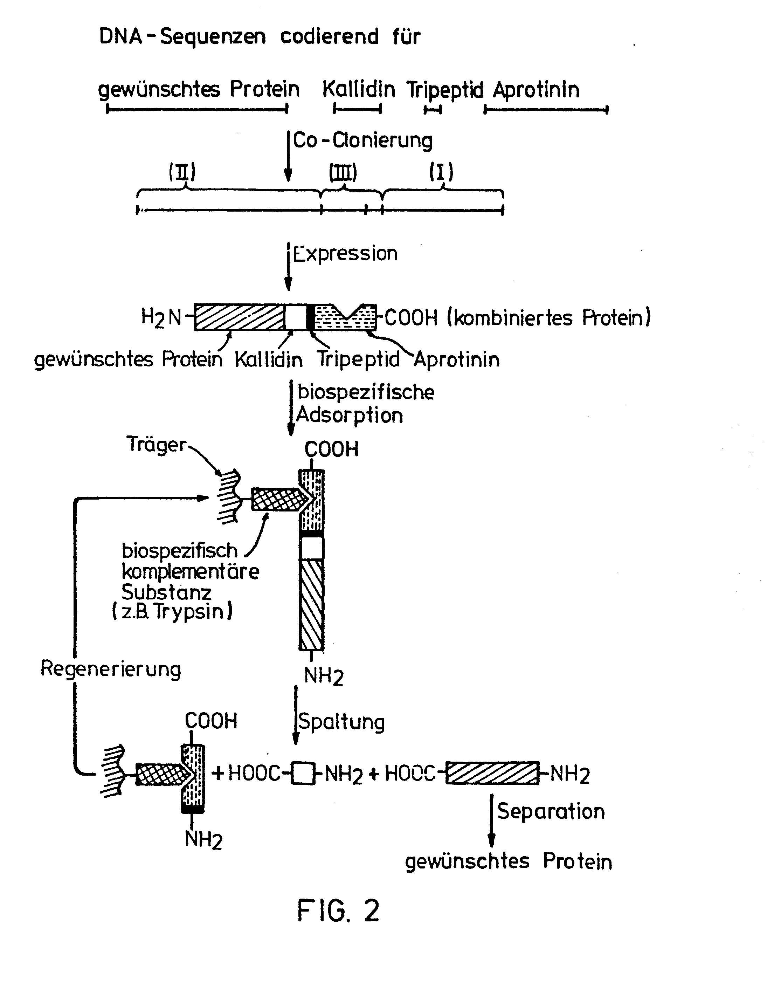

Die Fig. 1 und 2 veranschaulichen das erfindungsgemäße Verfahren in schematisierter Form.

Das beschriebene Verfahren ist auch möglich unter Verwendung eines 2er- oder 4er-Peptids anstelle des Tripeptids.

Das erfindungsgemäße Verfahren läßt sich, wie schon angesprochen, auch zur Herstellung des biospezifischen Proteins oder Polypeptids (I) einsetzen.

Diese Möglichkeit wird am Beispiel der Herstellung von Aprotinin näher erläutert.

Das Verfahren zur Herstellung von Aprotinin oder Aprotininderivaten besteht nun daraus, daß man

a)   eine DNA-Sequenz, die für Aprotinin oder ein Aprotinin-Derivat codiert,
     eine DNA-Sequenz, die für Kallidin codiert,
     eine DNA-Sequenz, die für ein Tripeptid $H-A_1'-A_2'-A_3'-OH$ codiert, vorzugsweise für H-Ser-Val-Gln-OH und
     eine DNA-Sequenz, die für ein Marker-Protein codiert
     in der Reihenfolge der Auflistung miteinander verknüpft;

b)   das so erhaltene neue DNA-Stück mittels geeigneter gentechnologischer Methoden so in die DNA eines Wirtsorganismus einbringt, daß die Expression eines kombinierten proteins erfolgen

Le A 23 922

kann, das aus mehreren Peptiden besteht, entsprechend den clonierten DNA-Sequenzen;

c) den Wirtsorganismus so kultiviert, daß er das kombinierte Protein erzeugt;

d) den das kombinierte Protein enthaltenden Teil des Kultivierungssystems, gegebenenfalls nach Abtrennen anderer Bestandteile, mit einem immobilisierten System in Kontakt bringt, das aus einem geeigneten Trägermaterial besteht, mit dem eine Substanz verbunden ist, die zu dem Aprotinin oder Aprotinin-Derivat komplementär ist und mit ihm unter den gegebenen Reaktionsbedingungen eine starke Bindung eingeht;

e) das immobilisierte System, an das das kombinierte Protein gebunden hat, in geeigneter Weise von übrigen Bestandteilen trennt und mit Kallikrein behandelt, das spezifisch das Kallidin aus dem gebundenen kombinierten Protein abspaltet;

f) die freigesetzten Bestandteile des kombinierten Proteins in geeigneter Weise von dem immobilisierten System, an das jetzt nur noch das Aprotinin bzw. Aprotinin-Derivat gebunden ist, trennt und

g) das Aprotinin oder Aprotinin-Derivat durch geeignete Elutionsmethoden vom immobilisierten System trennt und gewinnt.

0207402

Aprotinin und Aprotinin-Derivate können als unfusionierte Proteine in Zellen von E. coli nicht in größeren Mengen exprimiert werden. Eine mengenmäßig bedeutsame Expression wird erst in Form von Fusionsproteinen möglich, z.B. als C-terminale oder N-terminale Fusion mit einem Enzym, z.B. β-Galaktosidase. Nach der Fusion ergibt sich das Problem der Abtrennung des gewünschten Aprotinins vom nicht gewünschten Fusionspartner.

Eine direkte Fusion von Aprotinin an ein anderes Protein über Methionin als Bindestelle ist nicht praktikabel, da bei einer BrCN-Spaltung dieser Fusionsstelle auch das Aprotinin zerfällt, denn in Position 52 der Aminosäure-Sequenz des Aprotinins befindet sich ein Methionyl-Rest. Nur ein Aprotinin-Derivat, welches in Position 52 eine andere Aminosäure besitzt, wäre für die BrCN-Spaltungsmethode geeignet.

Die vorliegende Erfindung ermöglicht nun eine neue Herstellungsmethode für alle Aprotinin-Varianten. Durch die beschriebene Coclonierung des Aprotinins oder Aprotinin-Derivates mit anderen Peptiden entsteht ein Fusionspro-tein mit folgender Struktur:

Le A 23 922

Tripeptid

H$_2$N-|Aprotinin| -|Kallidin|-A$_1$'-A$_2$'-A$_3$'-Marker-Protein|-COOH

(oder Aprotinin-Derivat)

insbesondere

H$_2$N-|Aprotinin| - |Kallidin| - Ser-Val-Gln-|Marker-Protein|-COOH

(oder Aprotinin-Derivat)

Durch Herausschneiden des Dekapeptids Kallidin mit Hilfe des hochspezifischen Enzyms Kallikrein läßt sich das Fusionsprotein in drei Teilstücke zerlegen, von denen eines das gewünschte Aprotinin oder Aprotinin-Derivat ist.

Kallikrein spaltet sein natürliches Substrat - natives Kininogen - gleichzeitig an zwei Stellen mit hoher Umsatzrate unter Freisetzung des Dekapeptids Kallidin.

Le A 23 922

Kininogen-Sequenz

---Ser-Leu-Met-Lys-Arg-Pro-Pro-Gly-Phe-Ser-Pro-Phe-Arg-Ser-
          ↑                                   ↑

                                      Val-Gln----

⬇ Kallikrein

----Ser-Leu-Met-OH                H-Ser-Val-Gln----
        H-Lys-Arg-Pro-Pro-Gly-Phe-Ser-Pro-Phe-Arg-OH
        Kallidin (Dekapeptid, Intermediär-Peptid)

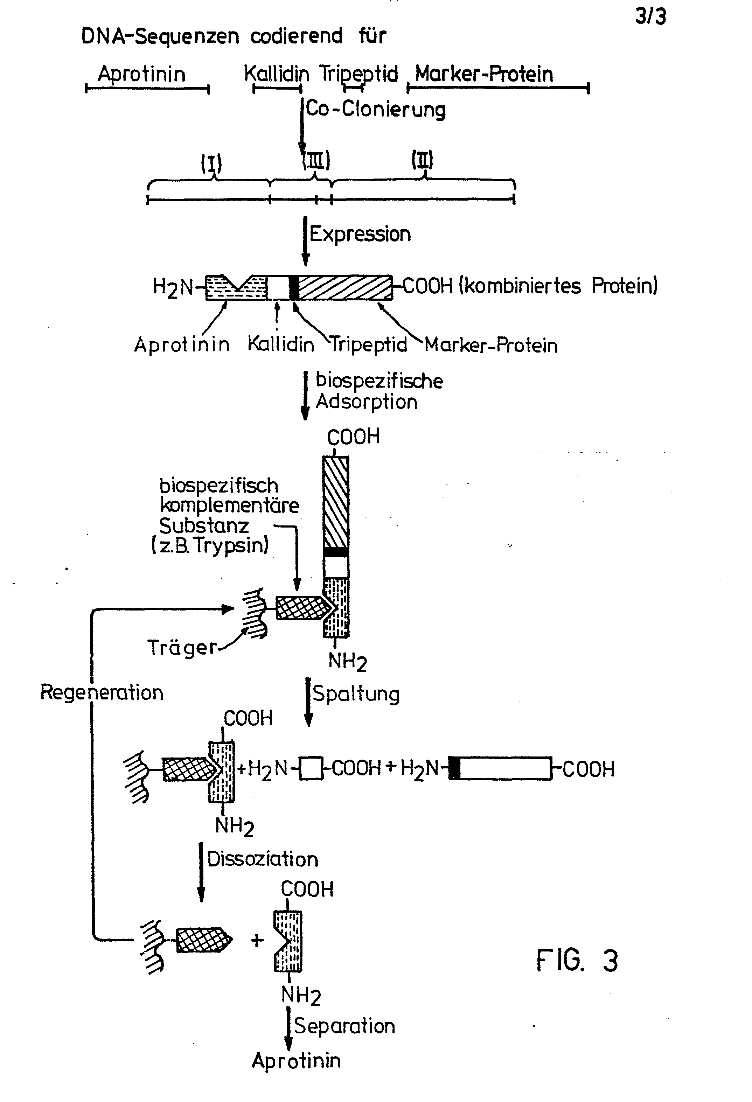

Die Fig. 3 zeigt das erfindungsgemäße Verfahren in schematisierter Form. Als Markerprotein wird normalerweise ein Enzym eingesetzt, welches durch seine Aktivität leicht nachweisbar ist. Beispielhaft seien hier genannt, die Alkalische Phosphatase, die β-Galaktosidase oder auch die Chloramphenicol-Acetyltransferase.

Als biospezifisch komplementäre Substanzen für die Isolation der Fusionsproteine können außer dem Trypsin auch andere Enzyme oder Proteine verwendet werden. Voraussetzung ist, daß sie einen Komplex bilden mit dem Aprotinin oder den in ihrer Aminosäuresequenz modifizierten Aprotininderivaten.

Patentansprüche

1. Verfahren zur Herstellung eines Proteins, wobei man

a) die DNA-Sequenz für ein biospezifisches Protein oder Polypeptid (I) und die DNA-Sequenz für ein weiteres Protein (II), welches das herzustellende Protein oder ein Markerprotein ist, über eine DNA-Sequenz,die ein kurzkettiges Peptid (III) codiert, welches als spezifische Spaltungsstelle dient, verknüpft.

b) das so erhaltene neue DNA-Stück mittels geeigneter gentechnologischer Methoden so in die DNA eines Wirtsorganismus einbringt, daß die Expression eines kombinierten Proteins erfolgen kann, das aus mehreren Peptiden besteht, entsprechend den clonierten DNA-Sequenzen;

c) den Wirtsorganismus so kultiviert, daß er das kombinierte Protein erzeugt;

d) den das kombinierte Protein enthaltenden Teil des Kultivierungssystems, gegebenenfalls nach Abtrennen anderer Bestandteile, mit einem immobilisierten System in Kontakt bringt, das aus einem geeigneten Trägermaterial besteht, mit dem eine Substanz verbunden ist, die zu dem biospezifischen Protein oder Polypeptid komplementär ist und mit ihm unter den gegebenen Reaktionsbedingungen eine starke Bindung eingeht;

Le A 23 922

e) das immobilisierte System, an das das kombinierte Protein gebunden hat, in geeigneter Weise von übrigen Bestandteilen trennt und mit Hilfe von einem oder mehreren Enzymen spezifisch spaltet,

f) das biospezifische Protein oder Polypeptid (I) von dem Protein (II) abtrennt und das jeweils gewünschte gewinnt,

dadurch gekennzeichnet, daß das kurzkettige Peptid (III) zusätzlich zur Spaltsequenz noch ein oder mehrere Aminosäuren enthält, die die Spaltungsspezifität und/oder die Spaltungsgeschwindigkeit erhöhen.

2. Verfahren nach Anspruch 1, dadurch gekennzeichnet, daß das herzustellende Protein das Protein (II) ist und daß die Bindung des biospezifischen Proteins oder Polypeptids (I) über das kurzkettige Peptid (III) derart erfolgt, daß die Spaltsequenz an das Protein (II) gebunden ist und die zusätzlichen Aminosäuren an das biospezifische Protein oder Polypeptid (I) gebunden sind.

3. Verfahren nach Anspruch 1, dadurch gekennzeichnet, daß das herzustellende Protein das biospezifische Protein oder Polypeptid (I) ist und das Protein (II) ein Markerprotein ist, wobei beide Proteine derart miteinander verbunden sind, daß die Spaltsequenz direkt an das biospezifische Protein oder Polypeptid (I) gebunden ist und die zusätzlichen Aminosäuren an das Markerprotein gebunden sind.

4. Verfahren nach den Ansprüchen 1 bis 3, dadurch gekennzeichnet, daß das kurzkettige Peptid (III) aus der Spaltsequenz und drei zusätzlichen Aminosäuren besteht, die entweder am C-Terminus oder am N-Terminus gebunden sind.

5. Verfahren nach den Ansprüchen 1 bis 4, dadurch gekennzeichnet, daß das spaltende Enzym das Kallikrein ist und das kurzkettige Peptid (III) aus Kallidin besteht, welches am C-Terminus die drei Aminosäuren mit der Sequenz H-Ser-Val-Gln-OH oder am N-Terminus die drei Aminosäuren mit der Sequenz H-Ser-Leu-Met-OH zusätzlich trägt. •

6. Verfahren nach den Ansprüchen 1 bis 3, dadurch gekennzeichnet, daß das biospezifische Protein oder Polypeptid (I) ein Enzym, ein Inhibitor, ein Hormon, ein Antikörper oder ein Rezeptor und die entsprechende dazu komplementäre Substanz an einem geeigneten Träger gebunden ist.

7. Verfahren nach den Ansprüchen 1 bis 3 und 6, dadurch gekennzeichnet, daß das biospezifische Protein oder Polypeptid (I) Aprotinin und die zu ihm komplementäre Substanz Trypsin oder Chymotrypsin ist.

8.  Verfahren nach den Ansprüchen 1 bis 3 und 6 dadurch gekennzeichnet, daß das biospezifische Protein oder Polypeptid (I) ein in seiner Biospezifität modifiziertes Aprotininderivat und die zu ihm komplementäre Substanz ein Enzym oder Protein ist.

9.  Verfahren nach den Ansprüchen 1 bis 3 und 7 und 8, dadurch gekennzeichnet, daß das biospezifische Protein oder Polypeptid (I) Aprotinin oder ein Aprotinin-Derivat und das Markerprotein ein Enzym ist.

10. Verfahren nach den Ansprüchen 1, 2 und 4 bis 8, dadurch gekennzeichnet, daß das Protein (II) ein Hormon, ein Plasmaprotein, ein Gerinnungsfaktor, ein Enzym, ein Antikörper oder eine Vakzine ist.

11. Verfahren nach Anspruch 10, dadurch gekennzeichnet, daß das herzustellende Protein Faktor VIII ist.

Le A 23 922

DNA-Sequenzen codierend für

FIG. 1

DNA-Sequenzen codierend für

gewünschtes Protein   Kallidin  Tripeptid  Aprotinin

↓ Co-Clonierung

(II)        (III)        (I)

↓ Expression

H₂N—[gewünschtes Protein | Kallidin | Tripeptid | Aprotinin]—COOH (kombiniertes Protein)

gewünschtes Protein  Kallidin  Tripeptid  Aprotinin

↓ biospezifische Adsorption

Träger

COOH

biospezifisch komplementäre Substanz (z.B. Trypsin)

Regenerierung

NH₂

↓ Spaltung

COOH

NH₂

+ HOOC—□—NH₂ + HOOC—[ ]—NH₂

↓ Separation

gewünschtes Protein

FIG. 2

FIG. 3